# EUROPÄISCHE PATENTSCHRIFT

(11) **EP 3 789 051 B1**
(45) Veröffentlichungstag und Bekanntmachung des Hinweises auf die Patenterteilung: **04.10.2023**
(21) Anmeldenummer: 20187288.4
(22) Anmeldetag: 23.07.2020
(51) Int. Cl.: A61M 1/00

(54) **AUFFANG-ANORDNUNG ZUM AUFNEHMEN VON ABGESOGENEM SEKRET, WELCHE EINEN FLÜSSIGKEITS-FILTER UND EINEN ERREGER-FILTER UMFASST**
CAPTURE ASSEMBLY FOR RECEIVING SIPHONED SECRETION COMPRISING A LIQUID FILTER AND A PATHOGEN FILTER
DISPOSITIF DE COLLECTE PERMETTANT DE RECEVOIR LA SÉCRÉTION EXTRAITE, COMPRENANT UN FILTRE DE LIQUIDE ET UN FILTRE D'AGENTS PATHOGÈNES

(30) Priorität: 04.09.2019 DE 102019006226
(43) Veröffentlichungstag der Anmeldung: 10.03.2021
(73) Patentinhaber: Drägerwerk AG & Co. KGaA, 23558 Lübeck (DE)
(72) Erfinder: Heyer, Harald, 23558 Lübeck (DE); Lokotsch, Heiko, 23558 Lübeck (DE); Reinboth, Thomas, 23558 Lübeck (DE)

(56) Entgegenhaltungen:
- WO-A1-2018/195101
- US-A1- 2017 072 119
- JIANQING LIU ET AL: "Triboelectric filtering for air purification", NANOTECHNOLOGY, INSTITUTE OF PHYSICS PUBLISHING, GB, Bd. 30, Nr. 29, 25. April 2019 (2019-04-25), Seite 292001, XP020341464, ISSN: 0957-4484, DOI: 10.1088/1361-6528/AB0E34 [gefunden am 2019-04-25]

## Beschreibung

Die Erfindung betrifft eine Auffang-Anordnung, welche Sekret aufzunehmen vermag, das aus einem Patienten abgesogen wird oder worden ist (siehe z.B. WO 2018/195101 A1).

Die Auffang-Anordnung umfasst eine Auffangeinheit. Um beispielsweise in einem Krankenhaus Sekret aus einem Patienten abzusaugen, wird eine Auffangeinheit einerseits mit einer patientenseitigen Koppeleinheit und andererseits mit einer Unterdruckquelle verbunden. In der Auffangeinheit wird ein Unterdruck erzeugt, und dadurch wird Sekret aus dem Körper des Patienten abgesogen und in die Auffangeinheit hinein gesogen und von dieser aufgenommen.

In DE 10 2009 035 353 A1 wird eine Vorrichtung zur Dränage von Flüssigkeit beschrieben. Ein Sammelbehälter 1 vermag Flüssigkeit aufzunehmen und besteht aus einem Behälteroberteil 2 und einem transparenten Behälterunterteil 3. Flüssigkeit 8 gelangt durch eine Einlassöffnung 5 in den Behälter 1, und eine Unterdruckquelle 7 vermag Luft durch eine Auslassöffnung 6 abzusaugen. In einem kastenförmigen Gehäuse 10 an der Innenseite des Behälteroberteils 2 ist ein Bakterienfilter 11 angeordnet. An der zur Innenseite zeigenden Seite des Gehäuses 10 ist ein Verschlusskörper 12 montiert, der als Hohlkörper in der Form eines hohlen Pyramidenstumpfs ausgestaltet ist und aus einem Sintermaterial besteht. Quellkörper 14 im Verschlusskörper 12 quellen bei Kontakt mit Flüssigkeit auf und verschließen dessen Poren.

In EP 1225930 B2 wird eine Auffang-Anordnung mit einem Umbehälter (collection container 1), einem Deckel (cover 4) und einem am Deckel hängenden Beutel (suction bag 3) beschrieben. Ein Vakuumanschluss (vacuum connector 2) ist über eine Leitung (conduit 10) am Umbehälter 1 befestigt. Eine Unterdruckquelle vermag über die Leitung 10 und eine weitere Leitung (conduit 7) einen Unterdruck im Behälter 3 herzustellen. An der Innenseite des Deckels 4 und an dem zum Behälter 3 zeigenden Ende der Leitung 7 ist ein Filter (filter 6) montiert. Falls dieser Filter 6 in Kontakt mit einer Flüssigkeit kommt, versiegelt der Filter 6 die Leitung 7.

Der Erfindung liegt die Aufgabe zugrunde, eine Auffang-Anordnung zum Aufnehmen von aus einem Patienten abgesogenem Sekret bereitzustellen, welche eine mit der Auffang-Anordnung verbundene Unterdruckquelle besser und / oder mit größerer Betriebssicherheit als bekannte Auffang-Anordnungen vor Verunreinigungen schützt.

Die Aufgabe wird durch eine Auffang-Anordnung mit den Merkmalen des Anspruchs 1 gelöst. Vorteilhafte Ausgestaltungen sind in den Unteransprüchen angegeben.

Die erfindungsgemäße Auffang-Anordnung vermag Sekret aufzunehmen, welches aus dem Körper eines Patienten abgesogen worden ist oder abgesogen wird. Die erfindungsgemäße Auffang-Anordnung umfasst
- eine Auffangeinheit,
- einen patientenseitigen Anschluss,
- einen Kanal,
- einen Flüssigkeits-Filter und
- einen Erreger-Filter mit mindestens einem triboelektrischen Vlies.

Die Auffangeinheit umschließt einen Auffangeinheit-Innenraum. In diesem Auffangeinheit-Innenraum vermag die Auffangeinheit abgesogenes Sekret aufzunehmen.

Der patientenseitige Anschluss ermöglicht es, eine patientenseitige Fluidverbindung herzustellen, um Sekret abzusaugen. Diese patientenseitige Fluidverbindung verbindet eine patientenseitige Kopplungseinheit mit dem Auffangeinheit-Innenraum. Diese patientenseitige Koppeleinheit ist außerhalb der Auffangeinheit angeordnet und lässt sich zeitweise mit einem Patienten verbinden. Die Auffangeinheit umfasst eine Wandung, welche eine Wand des Auffangeinheit-Innenraums bildet. Der Kanal führt durch diese Wandung hindurch. Bevorzugt bildet die Wandung einen Deckel der Auffangeinheit. Eine weitere Fluidverbindung, also eine Fluidverbindung zusätzlich zur patientenseitigen Fluidverbindung, lässt sich herstellen. Diese weitere Fluidverbindung, bevorzugt eine Unterdruck-Fluidverbindung, verbindet den Auffangeinheit-Innenraum mit einem von außen zugänglichen Anschlusspunkt und führt durch den Kanal hindurch.

Der Flüssigkeits-Filter ist fest mit der Wandung verbunden. Auch der Erreger-Filter ist fest mit der Wandung verbunden.

Der Erreger-Filter ist im Inneren der Wandung angeordnet und wird von der Wandung umgeben.

Der Flüssigkeits-Filter vermag folgende Funktion auszuführen:
- Wenn sich im Auffangeinheit-Innenraum eine Flüssigkeit befindet und der Spiegel dieser Flüssigkeit ansteigt und den Flüssigkeits-Filter erreicht, so vermag der Flüssigkeits-Filter wenigstens einen Teil dieser Flüssigkeit aufzusaugen.
- Als Folge hiervon verhindert der Flüssigkeits-Filter, dass diese Flüssigkeit in den Kanal eintritt. Zumindest vermag der Flüssigkeits-Filter das weitere Ansteigen der Flüssigkeit zu verzögern.

Der Erreger-Filter vermag folgende Funktion auszuführen:
- Er vermag aus einem Fluid welches aus dem Auffangeinheit-Innenraum herausströmt, sowohl Flüssigkeits-Tröpfchen als auch mikrobielle Erreger herauszufiltern, bevor diese den Kanal verlassen und durch den Anschlusspunkt austreten. Das ausströmende Fluid ist insbesondere ein Luftstrom mit Flüssigkeitströpfchen.

Erfindungsgemäß umfasst der Erreger-Filter mindestens ein triboelektrisches Vlies. Ein solches triboelektrische Vlies umfasst zwei zueinander parallelen Lagen aus zwei unterschiedlichen Faserarten, wobei ein Fluid zunächst durch die eine Lage und dann durch die andere Lage hindurch fließt. Diese beiden Lagen stehen senkrecht oder schräg auf der Durchströmrichtung und sind dergestalt miteinander verzahnt, dass die eine Lage wenigstens in einem Teil der Durchströmfläche, bevorzugt über ihre gesamte Ausdehnung, mechanisch in die andere Lage eindringt. Die eine Lage gibt Elektronen ab, und die andere Lage nimmt Elektronen auf oder zieht Elektronen an. Diese Elektronen vermögen Erreger zu binden und tragen dazu bei zu verhindern, dass Erreger das triboelektrische Vlies passieren können. Die aufnehmende Lage kann flussaufwärts oder flussabwärts von der abgebenden Lage angeordnet sein.

Ein triboelektrische Vlies hat insbesondere die folgenden Vorteile im Vergleich zu anderen Erreger-Filtern:
- Bei gleich großer Ausdehnung parallel zur Durchströmrichtung übt ein triboelektrisches Vlies einen geringeren Strömungswiderstand auf ein durchströmendes Fluid aus als andere Erreger-Filter, ohne dass die Filterleistung wesentlich geringer ist, oft sogar größer.
- Manche Erreger-Filter oder Partikel-Filter haben gesehen in eine Betrachtungsrichtung senkrecht auf die Durchströmrichtung eine Wellenform. Diese Wellenform erhöht die verfügbare Oberfläche des Filters. Im Vergleich zu einem solchen wellenförmigen Filter hat ein triboelektrische Filter eine geringere Abmessung in Durchströmrichtung. Dadurch lässt der triboelektrische Vlies sich in der Wandung der Auffang-Anordnung unterbringen, ohne dass eine große Kammer in oder an der Wandung vorgesehen werden muss.
- Ein triboelektrisches Vlies braucht nicht mit Chemikalien versehen zu werden, insbesondere nicht getränkt zu werden. Verglichen mit anderen Erreger-Filtern spart die Herstellung des triboelektrisches Vlieses einen Herstellungsschritt ein. Das triboelektrische Vlies weist keine Materialien auf, die für einen Menschen gefährlich sein können.
- Das triboelektrische Vlies kann nicht austrocknen und dadurch die gewünschte Filterfunktion im Laufe der Zeit verlieren.
- Verglichen mit manchen anderen möglichen Erreger-Filtern hat ein triboelektrisches Vlies eine erhöhte mechanische Stabilität gegenüber Belastung durch Zug und Druck.

Der von außen zugängliche Anschlusspunkt ist insbesondere ein geräteseitiger Anschluss, an den sich mittels der weiteren Fluidverbindung eine Unterdruckquelle anschließen lässt. Die weitere Fluidverbindung ist insbesondere eine Unterdruck-Fluidverbindung, welche einen Unterdruck im Inneren der Auffangeinheit herzustellen vermag. Das Fluid, welches durch den Kanal hindurchfließt und aus welchem der Erreger-Filter sowohl Flüssigkeits-Tröpfchen als auch mikrobielle Erreger herauszufiltern vermag, ist insbesondere ein Fluid zum Herstellen des Unterdrucks, fließt also aus dem Aufnahmeeinheit-Innenraum hinaus.

Die Erfindung ermöglicht es, zwei verschiedene Fluidverbindungen herzustellen.

Durch die patientenseitige Fluidverbindung hindurch wird Sekret aus dem Körper des Patienten abgesogen und gelangt in den Auffangeinheit-Innenraum, der von der Auffangeinheit umschlossen ist. Durch die weitere Fluidverbindung lässt sich ein Unterdruck im Inneren der Auffangeinheit herstellen, welcher das Absaugen des Sekrets durch die patientenseitige Fluidverbindung hindurch bewirkt. Die Auffangeinheit nimmt das abgesogene Sekret auf. Die weitere Fluidverbindung führt durch den Kanal in der Wandung hindurch.

Ermöglicht wird, die Auffang-Anordnung einmal zu verwenden, um in der Auffangeinheit abgesogenes Sekret aufzunehmen, und anschließend die Auffang-Anordnung mitsamt dem abgesogenen Sekret in der Auffangeinheit abzutransportieren, insbesondere zu entsorgen. Folgende weitere Bestandteile, die zum Absaugen des Sekrets benötigt werden oder sich verwenden lassen, können hingegen - gegebenenfalls nach einer geeigneten Reinigung - mehrmals verwendet werden:
- ein optionaler Umbehälter, der die erfindungsgemäße Auffang-Anordnung trägt und hält,
- eine patientenseitige Koppeleinheit, beispielsweise ein Katheter,
- eine fluiddichte Verbindung zwischen dem patientenseitigen Anschluss der Auffang-Anordnung und der patientenseitigen Koppeleinheit, bevorzugt mindestens einen Schlauch umfassend,
- eine Unterdruckquelle und
- eine fluiddichte Verbindung zwischen der Unterdruckquelle und dem Anschlusspunkt an der Auffang-Anordnung, bevorzugt mindestens einen weiteren Schlauch umfassend.

Verhindert werden muss, dass schädliche Substanzen aus dem Auffangeinheit-Innenraum durch den Anschlusspunkt hindurch austreten können, und zwar auch dann, wenn ein Unterdruck anliegt. Diese schädlichen Substanzen können mikrobielle Erreger, beispielsweise Bakterien und Viren, umfassen, welche in dem abgesogenen Sekret enthalten sind und sich daher im Auffangeinheit-Innenraum befinden. Das abgesogene Sekret enthält häufig außerdem Flüssigkeiten. Verhindert werden soll weiterhin, dass Flüssigkeit aus dem Auffangeinheit-Innenraum durch den Anschlusspunkt hinaus austreten kann, auch nicht Flüssigkeit in Tröpfchenform. Mikrobielle Erreger und Flüssigkeit, vor allem Flüssigkeits-Tröpfchen, können insbesondere eine angeschlossene Unterdruckquelle, welche ein Fluid aus dem Auffangeinheit-Innenraum absaugt, verunreinigen und / oder außer Betrieb setzen oder in die Umgebung gelangen.

Erfindungsgemäß vermag der Flüssigkeits-Filter Flüssigkeit aufzunehmen, welche den Flüssigkeits-Filter erreicht. Nach Aufnahme von Flüssigkeit vermag der Flüssigkeits-Filter den Kanal zu versperren oder wenigstens den Kanal-Durchmesser zu verringern. Bevorzugt dehnt ein Bestandteil des Flüssigkeits-Filters sich aus, wenn der Filter Flüssigkeit aufnimmt, und versperrt dadurch wenigstens teilweise den Kanal. Besonders bevorzugt enthält der Flüssigkeits-Filter eine Vielzahl von Poren und einen Stoff, der bei Kontakt mit einer Flüssigkeit, insbesondere Wasser, aufquillt. Durch das Aufquellen werden die Poren verschlossen. Weil der Flüssigkeits-Filter sich ausdehnt und / oder Poren verschlossen werden, vermag der Flüssigkeits-Filter zu verhindern, dass die Oberfläche von ansteigender Flüssigkeit im Auffangeinheit-Innenraum in den Kanal eintritt oder gar ansteigende Flüssigkeit durch den Anschlusspunkt hindurch austritt. Mindestens aber vermag der Flüssigkeits-Filter den Anstieg der Flüssigkeits-Oberfläche zu verzögern. Falls nur eine Verzögerung bewirkt wird, so steht mehr Zeit zur Verfügung, um den Anstieg der Flüssigkeit zu entdecken und durch eine weitere geeignete Maßnahme den weiteren Anstieg der Flüssigkeit zu verhindern, beispielsweise indem das Absaugen beendet oder der Unterdruck reduziert wird. Bevorzugt ist wenigstens ein Teil der Auffangeinheit aus einem durchsichtigen Material gefertigt, damit man von außen den Flüssigkeitsspiegel in den Auffangeinheit-Innenraum erkennen kann.

Erfindungsgemäß vermag der Erreger-Filter zu verhindern, dass mikrobielle Erreger und Flüssigkeits-Tröpfchen den Kanal passieren können. Dadurch vermag der Erreger-Filter insbesondere zu verhindern, dass Flüssigkeits-Tröpfchen oder Erreger im Anschlusspunkt aus der Auffangeinheit austreten. Erfindungsgemäß filtert der Erreger-Filter mikrobielle Erreger aus einem Strom von Fluid, der durch den Erreger-Filter hindurch fließt, heraus.

Die erfindungsgemäße Auffang-Anordnung filtert dank der beiden Filter sowohl Flüssigkeits-Tröpfchen als auch mikrobielle Erreger heraus, bevor diese den Anschlusspunkt erreichen. Die beiden Filter sind so angeordnet, dass sie auf ein Fluid wirken, welches in den Kanal eintritt und / oder durch den Kanal hindurchfließt.

Erfindungsgemäß sind die beiden Filter fest mit der Wandung verbunden, oder der Erreger-Filter ist im Inneren der Wandung angeordnet. Dadurch wird die Gefahr verringert, dass ein Filter während des Einsatzes verrutscht oder verschoben wird und dadurch in eine falsche Position gerät und deshalb Flüssigkeitstropfen oder Erreger den verschobenen Filter umgehen können. Weiterhin ist die Gefahr reduziert, dass ein Filter beim Transport zu einem Einsatzort oder während eines Einsatzes aus der Auffangeinheit rutscht und dadurch der Kanal frei ist.

Weiterhin lässt sich dank der Erfindung eine Auffang-Anordnung bereitstellen, welche bereits die beiden Filter in der jeweils richtigen Position umfasst. Nicht erforderlich ist es, vor einem Einsatz am Einsatzort einen Filter anzugeben. Insbesondere beim Einsatz einer Auffang-Anordnung in einem Krankenhaus ist es oft zeitaufwendig, im Krankenhaus selbst die erforderlichen Filter einzusetzen. Außerdem wird die Gefahr reduziert, dass ein Filter beim Einbau verunreinigt wird.

Weil die beiden Filter fest mit der Wandung verbunden sind oder der Erreger-Filter im Inneren der Wandung angeordnet ist, ist es nicht oder nur mit großem Aufwand möglich, die beiden Filter unerkannt oder gar zerstörungsfrei von der Wandung zu trennen. Dadurch wird insbesondere verhindert, dass jemand mindestens einen der beiden Filter von der Wandung trennt oder den Erreger-Filter aus der Wandung herausnimmt, bevor die Auffangeinheit mit der Wandung entsorgt wird, und diesen abgetrennten Filter in einer weiteren Auffangeinheit verwendet, um erneut Sekret abzusaugen. Diese Wiederverwendung widerspricht häufig hygienischen Bestimmungen und ist unerwünscht.

Der erfindungsgemäße Flüssigkeits-Filter vermag Flüssigkeit aufzusaugen, wenn diese den Flüssigkeits-Filter erreicht. Dadurch vermag der Flüssigkeits-Filter den weiteren Anstieg einer Flüssigkeit im Auffangeinheit-Innenraum zu verhindern oder wenigstens zu verzögern. Bevorzugt versperrt der Flüssigkeits-Filter den Kanal oder reduziert wenigstens den Kanal-Durchmesser, wenn der Flüssigkeits-Filter Flüssigkeit aufgesogen hat. Bevorzugt ragt der Flüssigkeits-Filter in den Auffangeinheit-Innenraum hinein. Die Positionierung des Flüssigkeits-Filters legt fest, wie weit Flüssigkeit im Auffangeinheit-Innenraum ansteigen kann, bis sie den Flüssigkeits-Filter erreicht und dann vom Flüssigkeits-Filter aufgesogen wird. Der Erreger-Filter vermag mikrobielle Erreger aus diesem Fluid herauszufiltern. Diese beiden Filter sind räumlich voneinander getrennt und grenzen aneinander an, oder gesehen in die Fließrichtung des Fluids tritt ein Abstand zwischen ihnen auf. Bevorzugt sind die beiden Filter fluiddicht und / oder partikeldicht miteinander verbunden.

Erfindungsgemäß sind zwei verschiedene Filter vorgesehen, die auf ihre jeweilige Aufgabe hin optimiert sind. Bevorzugt vermögen beide Filter Flüssigkeits-Tröpfchen aus einem Strom von Fluid herauszufiltern.

In der Regel steigt die Oberfläche einer Flüssigkeit im Aufnahmeeinheit-Innenraum an, wenn Sekret abgesogen wird. Erfindungsgemäß befindet der Erreger-Filter sich - gesehen in die Anstiegsrichtung - flussabwärts vom Flüssigkeits-Filter. Ein Fluid kann in eine Fließrichtung aus dem Aufnahmeeinheit-Innenraum heraus in den Kanal hinein und durch den Kanal hindurch fließen. Gesehen in diese Fließrichtung befindet sich der Erreger-Filter flussabwärts vom Flüssigkeits-Filter.

Dieses erfindungsgemäße Merkmal bewirkt einerseits, dass der Anstieg einer Flüssigkeit im Auffangeinheit-Innenraum beendet wird, bevor diese Flüssigkeit den Erreger-Filter erreicht, oder wenigstens bewirkt wird, dass die ansteigende Flüssigkeit später den Erreger-Filter erreicht. Insbesondere wird verhindert, dass der Erreger-Filter von Flüssigkeit umgeben wird. Dadurch wird die Gefahr reduziert, dass Flüssigkeit den Erreger-Filter erreicht und dessen Filterleistung reduziert.

Andererseits bewirkt dieses Merkmal in vielen Fällen, dass wenigstens ein Teil der Flüssigkeits-Tröpfchen aus dem Fluid herausgefiltert werden, bevor diese Tröpfchen mitsamt dem Fluid den Erreger-Filter erreichen. Diese Wirkung wird auch erzielt, bevor die Flüssigkeits-Oberfläche den Flüssigkeits-Filter erreicht. Weil Tröpfchen herausgefiltert werden, wird verhindert, dass der Erreger-Filter durch ansteigende Flüssigkeit oder durch Tröpfchen im Fluid verunreinigt und / oder außer Betrieb gesetzt wird. Der Flüssigkeits-Filter filtert alle oder wenigstens einen Teil der Flüssigkeits-Tröpfchen aus dem Fluid heraus, bevor diese den Erreger-Filter erreichen. Dadurch erreichen weniger oder sogar überhaupt keine Flüssigkeits-Tröpfchen den Erreger-Filter. Die Gefahr wird verringert, dass der Erreger-Filter durch Flüssigkeits-Tröpfchen funktionsunfähig wird. Die restlichen Flüssigkeits-Tröpfchen filtert der Erreger-Filter heraus. Der Erreger-Filter kann dank der Positionierung flussabwärts vom Flüssigkeits-Filter trotz Flüssigkeit im Aufnahmeeinheit-Innenraum sicher seine gewünschte Funktion ausüben.

Bevorzugt ist der Kanal vollständig oder wenigstens teilweise flussabwärts vom Erreger-Filter angeordnet. Diese Ausgestaltung reduziert weiter die Gefahr, dass ein Tröpfchen oder sonstige Partikel den Kanal erreichen und ihn verstopfen.

In einer Fortbildung dieser Ausgestaltung ist der Flüssigkeits-Filter an einer Innenseite der Wandung der Aufnahmeeinheit montiert. Diese Innenseite zeigt zum Auffangeinheit-Innenraum hin. Wenigstens ein Teil des Flüssigkeits-Filters ragt in den Auffangeinheit-Innenraum hinein. Diese Ausgestaltung ermöglicht es, dass der Flüssigkeits-Filter aufquillt, wenn er Flüssigkeit aufnimmt, bevor die Flüssigkeit denjenigen Bereich der Wandung erreicht, an dem der Flüssigkeits-Filter montiert ist, und durch das Aufquellen den Kanal versperrt. Das Aufquellen des Flüssigkeits-Filters wird dank dieser Position des Flüssigkeits-Filters nicht oder nur unwesentlich durch den Kanal oder durch die Wandung beeinträchtigt. Daher kann der Flüssigkeits-Filter stärker aufquellen und mehr Flüssigkeit aufnehmen und damit herausfiltern, als wenn der Flüssigkeits-Filter z.B. im Inneren des Kanals angeordnet wäre. Weil der Flüssigkeits-Filter fest an der Wandung montiert ist, wird die Gefahr verringert, dass er sich ungewollt von der Wandung löst. Außerdem behält er seine Position relativ zum Kanal und relativ zum Auffangeinheit-Innenraum bei. Weil der Flüssigkeits-Filter an der Innenseite der Wandung montiert ist, also zum Auffangeinheit-Innenraum hin zeigt, wird - verglichen mit einer denkbaren Montage an anderer Stelle - die Gefahr reduziert, dass der Flüssigkeits-Filter durch einen mechanischen Einfluss von außen umgeknickt oder abgebrochen wird und daher seine Funktion nicht mehr erfüllen kann.

Bevorzugt ist der Flüssigkeits-Filter an einem Deckel montiert, der zur Aufnahmeeinheit gehört und einen Behälter, z.B. einen dehnbaren Beutel, zum Aufnehmen von Sekret trägt. Der Kanal ist durch den Deckel hindurch geführt.

Besonders bevorzugt umfasst der Flüssigkeits-Filter einen Sinterfilter oder ist als ein Sinterfilter ausgestaltet. Dieser Sinterfilter vermag einerseits Flüssigkeit aufzusaugen, sobald diese Flüssigkeit den Sinterfilter erreicht, und quillt dann auf. Dadurch vermag der Sinterfilter den Anstieg von Flüssigkeit im Auffangeinheit-Innenraum zu verlangsamen oder sogar zu stoppen und auf diese Weise zu bewirken, dass die Flüssigkeit nicht oder wenigstens später den Erreger-Filter erreicht. Möglich ist, dass der aufquellende Sinterfilter nach Kontakt mit einer Flüssigkeit eine Auslassöffnung, die aus dem Auffangeinheit-Innenraum heraus führt, vollständig verschließt und auf diese Weise verhindert, dass Flüssigkeit den Erreger-Filter oder den Kanal erreicht. In vielen Fällen vermag der Sinterfilter andererseits Flüssigkeits-Tröpfchen aus einem Strom von Fluid herauszufiltern und abzuscheiden, bevor diese den Erreger-Filter erreichen.

Der Sinterfilter umfasst bevorzugt eine Vielzahl von Poren. In dem Sinterfilter ist weiterhin eine Substanz enthalten, welche bei Kontakt mit einer Flüssigkeit aufquillt und die Poren des Sinterfilters verstopft. Sobald eine Flüssigkeit im Auffangeinheit-Innenraum ansteigt und den Sinterfilter erreicht, so quillt diese Substanz auf und verschließt die Poren. Der Fluss von Fluid durch den Sinterfilter hindurch wird reduziert und - wenn das Aufquellen sich fortsetzt - schließlich ganz unterbunden. In der Regel sind die Poren bei vollständig aufgequollener Substanz vollständig verschlossen, sodass dann auch kein Gas durch den Sinterfilter mehr hindurchströmen kann.

Als Flüssigkeits-Filter lässt sich auch ein Filter aus einem anderen Material oder ein auf eine andere Weise hergestellter Filter verwenden, wobei das Material wenigstens vor dem Kontakt mit einer Flüssigkeit luftdurchlässig ist und Poren aufweist. Bevorzugt ist dieses Material wasserabweisend (hydrophob) oder ist mit einer wasserabweisenden Beschichtung versehen. Die Poren haben bevorzugt einen Durchmesser von jeweils 0,2 - 8 µm. Eine Flüssigkeit kann diesen Flüssigkeits-Filter erst dann durchdringen, wenn am Flüssigkeits-Filter ein bestimmter Mindestdruck anliegt. Dieser erforderliche Mindestdruck hängt vom Durchmesser der Poren, von der wasserabweisenden Eigenschaft sowie von dem Kontaktwinkel (Randwinkel, Benetzungswinkel) zwischen der Oberfläche des Filtermaterials und der Flüssigkeit ab. Der Unterdruck ist in der Regel durch die vor allem medizinischen Randbedingungen sowie durch Eigenschaften einer Unterdruckquelle gegeben. Aufgrund dieser Randbedingungen sowie der Geometrie der Auffangeinheit lassen sich ein geeignetes Material und optional eine geeignete wasserabweisende Beschichtung für den Flüssigkeits-Filter auswählen und verwenden.

Besonders bevorzugt hat der Flüssigkeits-Filter die Form eines Zylinders oder eines Kegelstumpfs, der sich zum Auffangeinheit-Innenraum hin verjüngt und in einer Ausgestaltung hohl ist.

Bevorzugt vermag der Flüssigkeits-Filter aus einem Strom von Fluid, welcher den Flüssigkeits-Filter durchfließt, wenigstens einen Teil von Flüssigkeits-Tröpfchen, die im Fluid enthalten sind, herauszufiltern, und zwar auch und insbesondere dann, wenn nicht eine Flüssigkeit den Flüssigkeits-Filter erreicht hat. Weniger Flüssigkeits-Tröpfchen erreichen den Erreger-Filter.

Bevorzugt ist der Erreger-Filter vollständig im Inneren der Wandung angeordnet. Die Wandung ist bevorzugt hohl, und der Erreger-Filter ist in diesem Innenraum im Inneren der Wandung angeordnet. Die Wandung, die um den Erreger-Filter herum angeordnet ist, schützt den Erreger-Filter vor mechanischen und chemischen Einflüssen, die von außen einwirken. Dadurch wird die Gefahr reduziert, dass der Erreger-Filter mechanisch beschädigt wird. Insbesondere wird das Risiko verringert, dass der Erreger-Filter einen Riss erhält, durch den mikrobielle Erreger hindurch fließen können und den Anschlusspunkt erreichen können.

Weiterhin wird die Gefahr reduziert oder sogar ausgeschlossen, dass jemand nach einem Einsatz den Erreger-Filter unerkannt aus der Auffangeinheit entnimmt und ihn erneut in einer anderen Auffangeinheit verwendet. Dies ist häufig aus hygienischen Gründen verboten und ist unerwünscht. Natürlich ist es theoretisch möglich, dass jemand die Wandung zerstört oder aufbricht und den Erreger-Filter entnimmt. Diese unerwünschte Entnahme ist aber mit erheblichem Aufwand verbunden und lässt sich leicht feststellen.

Bevorzugt vermag dieser Erreger-Filter aus einem Fluid, welche durch den Erreger-Filter hindurch fließt, zusätzlich Flüssigkeits-Tröpfchen herauszufiltern. Dadurch wird die Gefahr reduziert, dass Flüssigkeits-Tröpfchen den Anschlusspunkt erreichen. Denn diese müssten sowohl den Flüssigkeits-Filter als auch den Erreger-Filter passieren.

Erfindungsgemäß umfasst die Auffangeinheit ein triboelektrisches Vlies. Das triboelektrische Vlies enthält zwei Lagen aus zwei unterschiedlichen Faserarten. Bevorzugt sind die beiden Lagen aus jeweils einem Polymer hergestellt. Die eine Lage umfasst eine funktionelle Gruppe, die Elektronen abgeben, die eine Gruppe, die Elektronen aufnehmen oder ansaugen kann, z.B. ein Halogen.

In einer Ausgestaltung werden diese beiden Faserarten durch mechanisches Vernadeln dazu gebracht, sich gegenseitig zu durchdringen. Das Vernadeln wird beispielsweise mit Hilfe von Metallnadeln oder unter Hochdruck mit Wasserstrahlen durchgeführt. Bei diesem Vernadeln werden die beiden Lagen entgegengesetzt elektrisch aufgeladen.

Erfindungsgemäß ist der Erreger-Filter als ein triboelektrisches Vlies ausgestaltet. Dieses triboelektrische Vlies hat bevorzugt ein Flächengewicht zwischen 250 g/m2 und 500 g/m2. In einer Ausgestaltung umfasst der Erreger-Filter zusätzlich zu dem triboelektrisches Vlies ein Melt-Blown-Vlies.

Bevorzugt tritt ein Abstand zwischen dem Kanal, der zu dem Anschlusspunkt führt, und dem patientenseitigen Anschluss auf. Dadurch wird die Gefahr reduziert, dass abgesogenes Sekret nicht den Auffangeinheit-Innenraum erreicht, sondern direkt vom patientenseitigen Anschluss in den Kanal gelangt.

Im Folgenden wird die Erfindung anhand eines Ausführungsbeispiels beschrieben. Hierbei zeigen:
- Fig. 1: schematisch ein Auffang-System, welches eine Auffang-Anordnung, eine patientenseitige Kopplungseinheit und eine Unterdruckquelle umfasst;
- Fig. 2: in einer Querschnittsdarstellung die erfindungsgemäße Auffang-Anordnung mit den beiden Filtern.

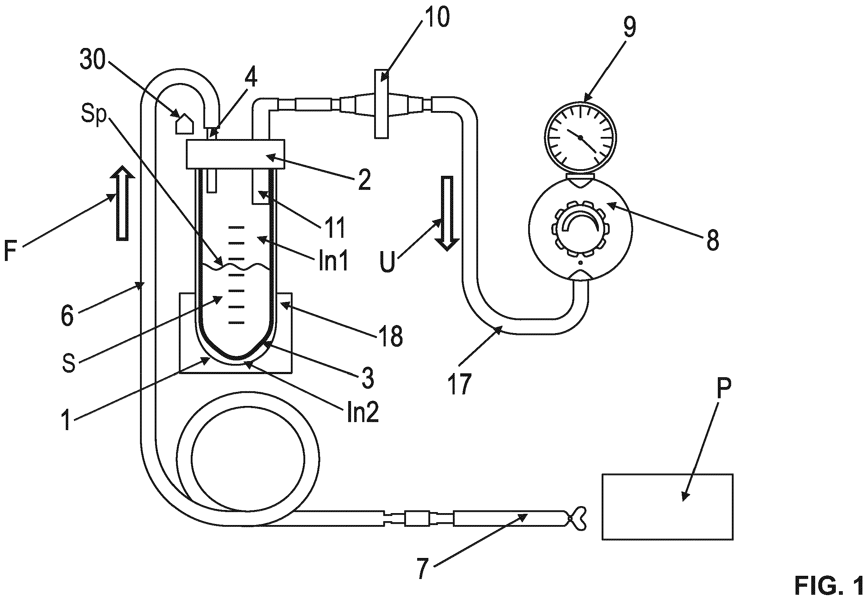

Figur 1 zeigt schematisch eine Absaug-Anordnung, welche ein Auffang-System mit einer erfindungsgemäßen Auffang-Anordnung umfasst. Das Auffang-System ist zeitweise mit einem Patienten P verbunden und vermag Sekret S aus dem Patienten P abzusaugen und aufzunehmen.

Die Auffang-Anordnung des Ausführungsbeispiels umfasst
- einen Deckel 2 mit einem umlaufenden Vorsprung 19, wobei der Deckel 2 dicht aber lösbar mit einem Umbehälter 1 verbunden ist,
- einen dehnbaren Beutel 3, der fluiddicht am Deckel 2 hängt,
- einen hohlen patientenseitigen Anschluss 4, der fest und fluiddicht mit dem Deckel 2 verbunden ist und nach außen hervorsteht, und
- eine Kappe 30, mit der sich der patientenseitige Anschluss 4 verschließen lässt.

Das Auffang-System umfasst diese Auffang-Anordnung 2, 3, 4, 30 und weiterhin
- einen Umbehälter 1, der fest mit einem Fuß 18 verbunden ist, welcher auf einem Untergrund steht, oder lösbar auf einen solchen Fuß 18 gestellt wird, wobei der dehnbarer Beutel 3 in den Umbehälter 1 hineinragt,
- einen Absaug-Schlauch 6, der sich auf den oder in den patientenseitigen Anschluss 4 aufsetzen oder in diesen einschrauben lässt, und
- einen geräteseitigen Anschluss 11 in einer Wand des Umbehälters 1.

Die Absaug-Anordnung umfasst das Auffang-System mit der erfindungsgemäßen Auffang-Anordnung und außerdem
- einen Katheter 7, der fluiddicht mit dem Absaug-Schlauch 6 verbunden ist und am Patienten P benutzt wird,
- eine Unterdruckquelle 8, die sich einschalten und ausschalten lässt,
- ein Differenzdruck-Messgerät 9, das denjenigen Unterdruck misst und anzeigt, welche die Unterdruckquelle 8 hergestellt hat,
- einen geräteseitigen Schlauch 17, der die Unterdruckquelle 8 fluiddicht mit dem geräteseitigen Anschluss 11 verbindet, und
- optional einen Filter 10 im Schlauch 17, der verhindert, dass Sekret-Tröpfchen in die Unterdruckquelle 8 gelangen.

Der Umbehälter 1 ist aus festem Kunststoff hergestellt, ist bevorzugt durchsichtig und wird mehrmals verwendet. Nach jeder Verwendung wird der Umbehälter 1 gereinigt.

Der Deckel 2 ist aus festem oder flexiblem Kunststoff. Bevorzugt sind seitlich am Deckel 2 ein oder zwei Griffe angeordnet, um den Deckel 2 öffnen und tragen zu können. Der Beutel 3 ist dehnbar, bevorzugt durchsichtig und bevorzugt an dem umlaufenden Vorsprung 19 befestigt. Der patientenseitige Anschluss 4 stellt einen Durchlass Ö zwischen der Außenseite des Deckels 2 und dem Auffangeinheit-Innenraum In1 im Beutel 3 bereit.

Der Beutel 3, der Deckel 2 und der patientenseitige Anschluss 4 gehören zur Auffangeinheit des Ausführungsbeispiels.

Der Deckel 2 und der dehnbare Beutel 3 umschließen zusammen einen Auffangeinheit-Innenraum In1. Zwischen der Außenwand des Beutels 3 und der Innenwand des Umbehälters 1 wird ein Innenraum In2 gebildet. Der patientenseitige Anschluss 4 stellt einen Durchlass Ö zwischen der Außenseite des Deckels 2 und dem Auffangeinheit-Innenraum In1 im Beutel 3 bereit. Der Absaug-Schlauch 6 lässt sich auf oder in den patientenseitigen Anschluss 4 aufsetzen oder aufschieben oder auch in den patientenseitigen Anschluss 4 einschrauben. Bei eingesetztem oder aufgesetztem oder eingeschraubtem Absaug-Schlauch 6 umschließt der Absaug-Schlauch 6 fluiddicht den patientenseitigen Anschluss 4, oder der Anschluss 4 umschließt fluiddicht den Schlauch 6. Die Kappe 30 lässt sich dann auf den patientenseitigen Anschluss 4 aufsetzen, wenn der Absaug-Schlauch 6 abgezogen oder auf andere Weise vom patientenseitigen Anschluss 4 entfernt ist.

Der geräteseitige Anschluss 11 fungiert im Ausführungsbeispiel als der Anschlusspunkt. In einer Ausgestaltung steht der geräteseitige Anschluss 11 in Fluidverbindung mit dem Innenraum In2. In einer bevorzugten Ausgestaltung steht hingegen auch der geräteseitige Anschluss 11 in Fluidverbindung mit dem Auffangeinheit-Innenraum In1. Durch das Innere des Deckels 2 ist ein Kanal 22 hindurch geführt, welcher bei eingesetztem Deckel 2 eine Fluidverbindung zwischen dem geräteseitigen Anschluss 11 und dem Auffangeinheit-Innenraum In1 herstellt, was weiter unten beschrieben wird.

In einer Ausgestaltung wird die gesamte Auffangeinheit 2, 3, 4 in einer geeigneten Verpackung zum Umbehälter 1 getragen, und dort werden die Bestandteile zum Auffang-System zusammengesetzt. Der Deckel 2, der patientenseitige Anschluss 4 und der gefüllte Beutel 3 werden einmal verwendet, wobei der Beutel 3 abgesogenes Sekret S aufnimmt, und werden anschließend aus dem Umbehälter 1 entnommen und mitsamt dem Sekret S entsorgt.

Die Absaug-Anordnung des Ausführungsbeispiels wird wie folgt betrieben, nachdem die Bestandteile so wie gerade beschrieben verbunden sind und der Katheter 7 gesetzt ist:
- Die Unterdruckquelle 8 wird eingeschaltet.
- Das Differenzdruck-Messgerät 9 misst den erzeugten Unterdruck und zeigt ihn an. Ein nicht gezeigtes Steuergerät oder auch eine überwachende Person stellen sicher, dass der erzeugte Unterdruck relativ zur Umgebung nicht größer als eine vorgegebene Schranke wird, also ein maximal zulässiger Unterdruck eingehalten wird.
- Luft wird aus dem Umbehälter 1 durch den Kanal 22 im Deckel 2 und den geräteseitigen Anschluss 11 hindurch und in Richtung U durch den geräteseitigen Schlauch 17 hindurch gesogen.
- In einer Ausgestaltung dehnt der im Umbehälter 1 erzeugte Unterdruck den Beutel 3, indem Luft aus dem Innenraum In2 abgesogen wird. In einer anderen Ausgestaltung wird Luft aus dem Beutel 3 durch den Kanal 22 im Deckel 2 hindurch abgesogen. In beiden Ausgestaltungen wird der Beutel 3 durch den Unterdruck gedehnt.
- Durch diesen Unterdruck im Beutel 3 wird bewirkt, dass Sekret S in Fließrichtung F aus dem Patienten P durch den Katheter 7, den Absaug-Schlauch 6, den patientenseitigen Anschluss 4 und den Kanal 22 im Deckel 2 hindurch in den Beutel 3 gesogen wird. Im Beutel 3 steigt der Spiegel Sp, also die Oberfläche des Sekrets S, im Beutel 3 an, solange weiteres Sekret S abgesogen wird.
- Sobald genügend Sekret S abgesogen ist oder der Beutel 3 bis zu einer vorgegebenen Schranke gefüllt ist, wird der Absaug-Schlauch 6 vom patientenseitigen Anschluss 4 abgezogen. Dadurch wird das Absaugen beendet. Die Unterdruckquelle 8 bleibt zunächst eingeschaltet, um den elastischen Beutel 3 gedehnt zu halten.
- Die Kappe 30 wird auf den patientenseitigen Anschluss 4 aufgesetzt.
- Nunmehr wird die Unterdruckquelle 8 abgeschaltet. In einer Ausgestaltung ist es nicht erforderlich, den geräteseitigen Schlauch 17 vom geräteseitigen Anschluss 11 am Umbehälter 1 abzuziehen.
- Die Auffangeinheit mit dem gefüllten Beutel 3, dem Deckel 2 und dem mittels der Kappe 30 verschlossenen patientenseitigen Anschluss 4 wird aus dem Umbehälter 1 entnommen und entsorgt.
- Der Umbehälter 1 wird gereinigt und steht für einen neuen Einsatz zur Verfügung.

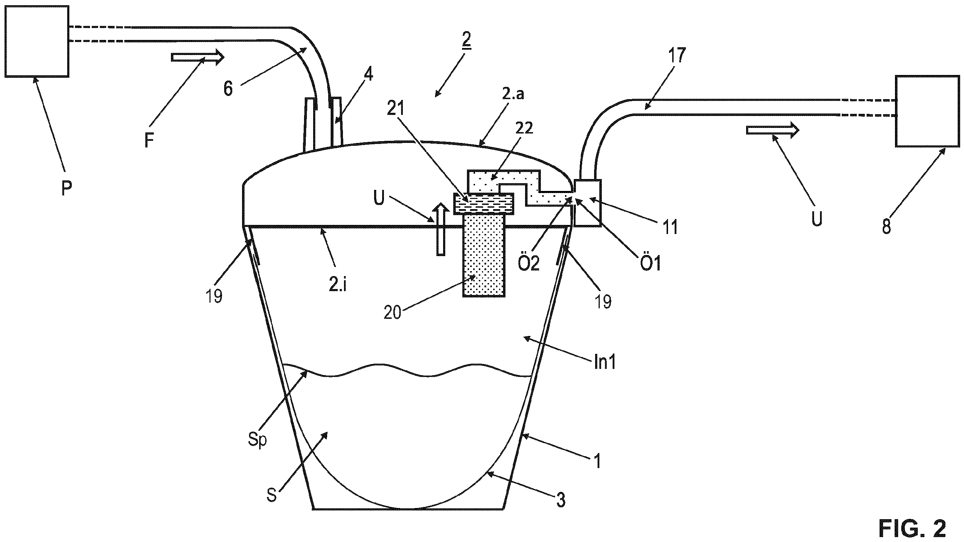

Figur 2 zeigt in einer Querschnittsdarstellung die erfindungsgemäße Auffang-Anordnung. Gleiche Bestandteile haben dieselben Bezugszeichen wie in Figur 1. Gezeigt werden folgende zusätzlichen Bestandteile:
- die gewölbte Außenseite 2.a des Deckels 2,
- die zum Auffangeinheit-Innenraum In1 hin zeigende und bevorzugt ebene Innenseite 2.i des Deckels 2,
- ein zylinderförmiger Sinterfilter 20, der als der Flüssigkeits-Filter fungiert, an der Innenseite 2.i des Deckels 2 montiert ist und in den Auffangeinheit-Innenraum In1 hineinragt,
- ein Vlies 21, welches als der Erreger-Filter fungiert, im Inneren des Deckels 2 montiert ist und bevorzugt partikeldicht mit dem Sinterfilter 20 verbunden ist,
- der Kanal 22, der durch den Deckel 2 hindurch geführt ist, bevorzugt partikeldicht mit dem Vlies 21 verbunden ist und punktiert dargestellt ist,
- eine Öffnung Ö2 in der Wand des Deckels 2, wobei der Kanal 22 in der Öffnung Ö2 endet, und
- eine Öffnung Ö1 in dem geräteseitigen Anschluss 11.

Falls der Deckel 2 korrekt auf den Umbehälter 1 aufgesetzt ist, überlappen bevorzugt die Öffnungen Ö1 und Ö2 wenigstens teilweise miteinander. Bevorzugt stellen nicht gezeigte Führungselemente sicher, dass der Deckel 2 korrekt in den Umbehälter 1 eingesetzt wird und daher die Öffnungen Ö1 und Ö2 wenigstens teilweise überlappen.

Sobald die Unterdruckquelle 8 eingeschaltet ist, wird Luft durch den geräteseitigen Schlauch 17 in die Richtung U aus der Auffangeinheit 2, 3, 4 abgesogen. Der geräteseitige Schlauch 17 steht über den geräteseitigen Anschluss 11, die beiden Öffnungen Ö1 und Ö2, den Kanal 22, das Vlies 21 und den Sinterfilter 20 in einer Fluidverbindung mit dem Auffangeinheit-Innenraum In1. Diese Fluidverbindung ist so ausgestaltet, dass kein Fluid in einem Bypass aus dem Beutel 3 gesogen werden kann, also die beiden Filter 20 und 21 umgehen kann. Vielmehr muss abgesogenes Fluid stets beide Filter 20, 21 passieren.

Der Sinterfilter 20 des Ausführungsbeispiels umfasst mehrere Quellkörper, die - bevorzugt durch Sintern - zu einem festen Bauteil verbunden sind und zwischen denen Poren mit unterschiedlichen Abmessungen auftreten, bevorzugt mit Porengrößen zwischen 18 und 65 µm. Bei Kontakt mit einer Flüssigkeit, insbesondere mit dem Flüssigkeitsspiegel Sp oder mit Flüssigkeits-Tröpfchen, saugen die Quellkörper die Flüssigkeit auf, quellen auf und verstopfen nach und nach die Poren. Falls die Flüssigkeit durch medizinisches Absaugen aus einem Patienten erzeugt wird, quillt der Sinterfilter 20 irreversibel auf. Weil sie aufgesogen wird, kann Flüssigkeit nicht den aufgequollenen Sinterfilter 20 passieren. Sobald die Quellkörper ausreichend aufgequollen sind, verstopfen sie vollständig die Poren, sodass überhaupt kein Fluid, auch kein Gas, mehr durch den Sinterfilter 20 fließen kann. Das irreversible Aufquellen verhindert, dass Flüssigkeit in den Kanal 22 eindringen kann, und zeigt an, dass der Sinterfilter 20 benutzt wurde, und verhindert eine unerkannte Wiederverwendung.

Das Vlies 21 ist in einer Realisierung als triboelektrisches Vlies ausgestaltet und hat bevorzugt ein Flächengewicht zwischen 250 g/m2 und 500 g/m2. In einer anderen Realisierung umfasst es zusätzlich einen Melt-Blown-Filter ausgestaltet und weist bevorzugt ein Flächengewicht zwischen 20 g/m² bis 100 g/m2 auf. Möglich ist, dass das Vlies mindestens zwei Lagen aufweist, die senkrecht oder schräg auf der Durchfließrichtung stehen, beispielsweise drei Lagen mit einem Flächengewicht von 20 g/m². Die Lagen können aus unterschiedlichen Materialien aufgebaut sein.

Der Sinterfilter 20 beendet den Anstieg von Flüssigkeit im Auffangeinheit-Innenraum In1, sobald der ansteigende Spiegel Sp den Sinterfilter 20 erreicht hat, oder verlangsamt ihn zumindest. Dadurch steht mehr Zeit zur Verfügung, um die Unterdruckquelle 8 rechtzeitig auszuschalten und dadurch das Absaugen zu beenden. Außerdem filtert der Sinterfilter 20wenigstens einen Teil der Flüssigkeits-Tröpfchen aus dem Strom von Fluid heraus, welche aus dem Beutel 3 gesogen wird. Dadurch erreichen weniger oder sogar gar keine Flüssigkeits-Tröpfchen mehr den Vlies 21, und der Vlies 21 ist weitgehend vor Feuchtigkeit geschützt.

Das Vlies 21 ist flussabwärts vom Sinterfilter 20 angeordnet und filtert mikrobielle Erreger aus dem abgesogenen Strom von Fluid heraus. Außerdem filtert das Vlies 21 diejenigen Flüssigkeits-Tröpfchen aus dem Strom von Fluid heraus, welche der Sinterfilter 20 nicht herausgefiltert hat. Dadurch verhindert das Vlies 21, dass diese Flüssigkeits-Tröpfchen den Kanal 22 erreichen. Das Vlies 21 ist vollständig vom Deckel 2 umgeben und dadurch vor mechanischen Beschädigungen und Druckbeaufschlagung geschützt. Außerdem ist es nicht möglich, das Vlies 21 zu entfernen, ohne den Deckel 2 zu zerstören. Somit lässt das Vlies 21 sich nicht unerkannt entnehmen und in einem anderen Deckel erneut verwenden.

Der Sinterfilter 20 und das Vlies 21 sind fest mit den Deckel 2 verbunden. Die beiden Filter 20, 21 lassen sich nicht unbemerkt entfernen. Um das Vlies 21 zu entfernen, müsste der Deckel 2 stellenweise aufgebrochen werden.

**BEZUGSZEICHENLISTE**

| | |
|---|---|
| 1 | Umbehälter, nimmt den Beutel 3 auf, trägt den geräteseitigen Anschluss 11, gehört zum Auffang-System |
| 2 | Deckel für den Umbehälter 1, nimmt den patientenseitigen Anschluss 4 auf, gehört zur Auffangeinheit |
| 2.a | gewölbte Außenseite des Deckels 2 |
| 2.i | zum Auffangeinheit-Innenraum In1 hin zeigende ebene Innenseite des Deckels 2 |
| 3 | dehnbarer Beutel, nimmt Sekret S vom Patienten P auf, vom Deckel 2 im Inneren des Umbehälters 1 gehalten, trägt den Beutel 3, gehört zur Auffangeinheit |
| 4 | patientenseitiger Anschluss, in den Deckel 2 integriert, stellt einen Durchlass zwischen der Außenseite des Deckels 2 und dem Auffangeinheit-Innenraum In1 bereit |
| 6 | Absaug-Schlauch, gehört zur patientenseitigen Kopplungseinheit, mit dem patientenseitigen Anschluss 4 und mit dem Katheter 7verbunden |
| 7 | Katheter, mit dem Absaug-Schlauch 6 verbunden, gehört zur patientenseitigen Kopplungseinheit |
| 8 | Unterdruckquelle, umfasst eine Pumpe, über den geräteseitigen Schlauch 17 mit dem geräteseitigen Anschluss 11 verbunden |
| 9 | Differenzdruck-Messgerät, welche den von der Unterdruckquelle 8 erzeugten Unterdruck misst und anzeigt |
| 10 | optionaler Filter zwischen dem geräteseitigen Anschluss 11 und der Unterdruckquelle 8, im geräteseitigen Schlauch 17 angeordnet |
| 11 | geräteseitiger Anschluss, am Umbehälter 1 angeordnet, lässt sich über den geräteseitigen Schlauch 17 mit der Unterdruckquelle 8 verbinden, fungiert als Anschlusspunkt |
| 17 | geräteseitiger Schlauch, verbindet den geräteseitigen Anschluss 11 mit der Unterdruckquelle 8 |
| 18 | Fuß, auf dem der Umbehälter 1 steht |
| 19 | umlaufender Vorsprung innen am Deckel 2, trägt den Beutel 3, mit dem Umbehälter 1 verbunden |
| 20 | zylinderförmiger Sinterfilter, an der Innenseite des Deckels 2 montiert, ragt in den Beutel 3 hinein, fungiert als Flüssigkeits-Filter |
| 21 | Vlies, im Kanal 22 angeordnet, partikeldicht mit dem Sinterfilter 20 verbunden, als triboelektrisches Vlies und optional zusätzlich mit einem Melt-Blown-Vlies ausgestaltet, fungiert als Erreger-Filter |
| 22 | Kanal, der die Öffnung Ö2 mit dem Auffangeinheit-Innenraum In1 verbindet, beginnt im Vlies 21 |
| F | Flussrichtung von Sekret durch den Absaug-Schlauch 6 in den Beutel 3 |
| In1 | Innenraum im Beutel 3, steht über den Kanal 22 in Fluidverbindung mit der Öffnung Ö2 |
| In2 | Raum zwischen dem Beutel 3 und der Innenwand des Umbehälters 1 |
| P | Patient, mit dem Katheter 7 verbunden |
| Ö1 | Öffnung im geräteseitigen Anschluss 11, überlappt bei korrektem Sitz des Deckels 2 bevorzugt fluiddicht mit der Öffnung Ö2 |
| Ö2 | Öffnung im Deckel 2, bildet ein Ende des Kanals 22, überlappt bei korrektem Sitz des Deckels 2 bevorzugt fluiddicht mit der Öffnung Ö1 |
| S | vom Patienten P abgesogenes und im Beutel 3 gesammeltes Sekret |
| Sp | Flüssigkeits-Spiegel des abgesogenen Sekrets S im Beutel 3 |
| U | Flussrichtung von Luft durch den geräteseitigen Schlauch 17 |

## Patentansprüche

1. Auffang-Anordnung zum Aufnehmen von aus einem Patienten (P) abgesogenem Sekret (S),
wobei die Auffang-Anordnung
- eine Auffangeinheit (2, 3) mit einer Wandung (2),
- einen patientenseitigen Anschluss (4),
- einen Kanal (22),
- einen fest mit der Wandung (2) verbundenen und / oder im Inneren der Wandung (2) angeordneten Flüssigkeits-Filter (20) und
- einen fest mit der Wandung (2) verbundenen Erreger-Filter (21) umfasst,
wobei der Erreger-Filter (21) vollständig im Inneren der Wandung (2) angeordnet ist und vollständig von der Wandung (2) umgeben ist,
wobei die Auffangeinheit (2, 3)
- einen Auffangeinheit-Innenraum (In1) umschließt und
- zum Aufnehmen von abgesogenem Sekret (S) im Auffangeinheit-Innenraum (In1) ausgestaltet ist,
wobei der patientenseitige Anschluss (4) die Herstellung einer patientenseitigen Fluidverbindung zwischen einer patientenseitigen Kopplungseinheit (6, 7) außerhalb der Auffangeinheit (2, 3) und dem Auffangeinheit-Innenraum (In1) ermöglicht,
wobei der Kanal (22)
- durch die Wandung (2) hindurch führt und
- die Herstellung einer weiteren Fluidverbindung zwischen einem von außen zugänglichen Anschlusspunkt, insbesondere einem geräteseitigen Anschluss (11), und dem Auffangeinheit-Innenraum (In1) ermöglicht und
wobei der Flüssigkeits-Filter (20) dazu ausgestaltet ist, wenigstens einen Teil einer Flüssigkeit aufzusaugen, welche im Inneren des Auffangeinheit-Innenraum (In1) ansteigt, sobald diese den Flüssigkeits-Filter (20) erreicht, und
wobei der Erreger-Filter (21)
- dazu ausgestaltet ist, aus einem Fluid, welches aus dem Auffangeinheit-Innenraum (In1) herausfließt, mikrobielle Erreger und Flüssigkeits-Tröpfchen herauszufiltern, bevor diese den Anschlusspunkt (11) erreichen, und
- mindestens ein triboelektrisches Vlies umfasst,
wobei - gesehen in die Fließrichtung (U) von Fluid aus dem Aufnahmeeinheit-Innenraum (In1) hinaus - der Erreger-Filter (21) flussabwärts vom Flüssigkeits-Filter (20) angeordnet ist,
wobei das oder jedes triboelektrische Vlies zwei zueinander parallele Lagen aus zwei unterschiedlichen Faserarten umfasst,
wobei diese beiden Lagen
- senkrecht oder schräg auf der Durchströmrichtung stehen und
- dergestalt miteinander verzahnt sind, dass die eine Lage wenigstens in einem Teil der Durchströmfläche mechanisch in die andere Lage eindringt, und
wobei die eine Lage Elektronen abgibt und die andere Lage Elektronen aufnimmt oder Elektronen anzieht.

2. Auffang-Anordnung nach Anspruch 1,
**dadurch gekennzeichnet, dass**
der Flüssigkeits-Filter (20) an einer zum Auffangeinheit-Innenraum (In1) hin zeigenden Innenseite (2.i) der Wandung (2) montiert ist.

3. Auffang-Anordnung nach Anspruch 1 oder Anspruch 2,
**dadurch gekennzeichnet, dass**
die beiden Lagen aus den beiden unterschiedlichen Faserarten sich gegenseitig durchdringen,
wobei das gegenseitige Durchdringen durch mechanisches Vernadeln hergestellt ist und
wobei die beiden Lagen durch das Vernadeln entgegengesetzt elektrisch aufgeladen sind.

4. Auffang-Anordnung nach einen der vorhergehenden Ansprüche,
**dadurch gekennzeichnet, dass**
der Flüssigkeits-Filter (20) einen Sinterfilter umfasst.

5. Auffang-Anordnung nach einen der vorhergehenden Ansprüche,
**dadurch gekennzeichnet, dass**
der Erreger-Filter (21) zusätzlich ein Melt-Blown-Vlies umfasst.

6. Auffang-Anordnung nach einen der vorhergehenden Ansprüche,
**dadurch gekennzeichnet, dass**
der Flüssigkeits-Filter (20) dazu ausgestaltet ist, wenigstens einen Teil einer Flüssigkeit aufzusaugen, welche im Inneren des Auffangeinheit-Innenraum (In1) den Flüssigkeits-Filter (20) erreicht, und
zusätzlich dazu ausgestaltet ist, Flüssigkeits-Tropfen aus einem Strom von Fluid heraus zu saugen, welche durch den Flüssigkeits-Filter (20) hindurchfließt.

7. Auffang-Anordnung nach einen der vorhergehenden Ansprüche,
**dadurch gekennzeichnet, dass**
der Kanal (22) von dem patientenseitigen Anschluss (4) beabstandet angeordnet ist.

8. Auffang-System umfassend
- eine Auffang-Anordnung nach einem der vorhergehenden Ansprüche,
- einen Umbehälter (1) und
- einen geräteseitigen Anschluss (11),
wobei der Umbehälter (1) die Auffangeinheit (2, 3, 4) aufnimmt und trägt,
wobei der geräteseitige Anschluss (11) die Herstellung einer Unterdruck-Fluidverbindung zwischen dem Auffangeinheit-Innenraum (In1) und einer Unterdruckquelle (8) außerhalb des Umbehälters (1) ermöglicht und
wobei die Unterdruck-Fluidverbindung durch den Kanal (22) hindurch führt.

9. Absaug-Anordnung umfassend
- eine Unterdruckquelle (8),
- eine patientenseitige Kopplungseinheit (6, 7) und
- ein Auffang-System nach Anspruch 8,
wobei die patientenseitige Kopplungseinheit (6, 7) fluiddicht mit dem patientenseitigen Anschluss (4) verbunden ist,
wobei die Unterdruckquelle (8) fluiddicht mit dem geräteseitigen Anschluss (11) verbunden ist,
wobei eine patientenseitige Fluidverbindung
- zwischen der patientenseitigen Kopplungseinheit (6, 7) und dem Auffangeinheit-Innenraum (In1) hergestellt oder herstellbar ist und
- durch den patientenseitigen Anschluss (4) führt und
wobei eine Unterdruck-Fluidverbindung
- zwischen der Unterdruckquelle (8) und dem Auffangeinheit-Innenraum (In1) hergestellt oder herstellbar ist und
- durch den geräteseitigen Anschluss (11) und durch den Kanal (22) hindurch führt.

## Claims

1. Collecting assembly for receiving secretion (S) aspirated from a patient (P),
the collecting assembly comprising
- a collecting unit (2, 3) with a wall (2),
- a patient-side connector (4),
- a duct (22),
- a liquid filter (20) which is connected fixedly to the wall (2) and/or is arranged in the interior of the wall (2), and
- a pathogen filter (21) which is connected fixedly to the wall (2),
the pathogen filter (21) being arranged completely in the interior of the wall (2) and being surrounded completely by the wall (2),
the collecting unit (2, 3)
- enclosing a collecting unit interior space (In1) and
- being configured to receive aspirated secretion (S) in the collecting unit interior space (In1),
the patient-side connector (4) enabling the establishing of a patient-side fluid connection between a patient-side coupling unit (6, 7) outside the collecting unit (2, 3) and the collecting unit interior space (In1),
the duct (22)
- leading through the wall (2) and
- enabling the establishing of a further fluid connection between a connector point accessible from the outside, in particular a unit-side connector (11), and the collecting unit interior space (In1), and
the liquid filter (20) being configured to absorb at least part of a liquid which rises in the interior of the collecting unit interior space (In1) as soon as this liquid reaches the liquid filter (20), and
the pathogen filter (21)
- being configured to filter microbial pathogens and liquid droplets from a fluid which flows out of the collecting unit interior space (In1) before they reach the connector point (11), and
- comprises at least one tribo-electrical nonwoven,
the pathogen filter (21) being arranged downstream of the liquid filter (20), as viewed in the direction of flow (U) of fluid out of the receiving unit interior space (In1),
the or each tribo-electrical nonwoven comprising two mutually parallel layers consisting of two different fibre types,
these two layers
- lying perpendicularly or obliquely on the throughflow direction and
- being interlocked with one another in such a way that the one layer penetrates mechanically into the other layer at least in part of the throughflow area, and
the one layer discharging electrodes and the other layer absorbing electrons or attracting electrons.

2. Collecting assembly according to Claim 1,
**characterized in that**
the liquid filter (20) is mounted on an inner side (2.i) of the wall (2) pointing towards the collecting unit interior space (In1).

3. Collecting assembly according to Claim 1 or Claim 2,
**characterized in that**
the two layers consisting of the two different fibre types penetrate one another mutually,
the mutual penetration being produced by way of mechanical needling, and the two layers being electrically charged with opposite signs by way of the needling.

4. Collecting assembly according to one of the preceding claims,
**characterized in that**
the liquid filter (20) comprises a sintered filter.

5. Collecting assembly according to one of the preceding claims,
**characterized in that**
the pathogen filter (21) additionally comprises a meltblown nonwoven.

6. Collecting assembly according to one of the preceding claims,
**characterized in that**
the liquid filter (20) is configured to absorb at least part of a liquid which reaches the liquid filter (20) in the interior of the collecting unit interior space (In1), and
is additionally configured to suck liquid drops out of a flow of fluid which flows through the liquid filter (20).

7. Collecting assembly according to one of the preceding claims, **characterized in that**
the duct (22) is arranged spaced apart from the patient-side connector (4).

8. Collecting system comprising
- a collecting assembly according to one of the preceding claims,
- a surrounding container (1) and
- a unit-side connector (11),
the surrounding container (1) receiving and supporting the collecting unit (2, 3, 4),
the unit-side connector (11) enabling the establishing of a vacuum fluid connection between the collecting unit interior space (In1) and a vacuum source (8) outside the surrounding container (1), and
the vacuum fluid connection leading through the duct (22).

9. Aspirating assembly comprising
- a vacuum source (8),
- a patient-side coupling unit (6, 7) and
- a collecting system according to Claim 8,
the patient-side coupling unit (6, 7) being connected in a fluid-tight manner to the patient-side connector (4),
the vacuum source (8) being connected in a fluid-tight manner to the unit-side connector (11),
a patient-side fluid connection
- being established or being capable of being established between the patient-side coupling unit (6, 7) and the collecting unit interior space (In1) and
- leading through the patient-side connector (4), and
a vacuum fluid connection
- being established or being capable of being established between the vacuum source (8) and the collecting unit interior space (In1) and
- leading through the unit-side connector (11) and through the duct (22).

## Revendications

1. Dispositif de collecte conçu pour recevoir des sécrétions (S) évacuées d'un patient (P) par aspiration,
ledit dispositif de collecte comprenant
- une unité collectrice (2, 3) munie d'une paroi (2),
- un raccord (4) situé côté patient,
- un canal (22),
- un filtre (20) à liquides, relié rigidement à la paroi (2) et/ou intégré dans l'espace interne de ladite paroi (2) et
- un filtre (21) à agents pathogènes, relié rigidement à ladite paroi (2), lequel filtre (21) à agents pathogènes est intégré en totalité dans l'espace interne de la paroi (2) et est intégralement entouré par ladite paroi (2),
sachant que l'unité collectrice (2, 3)
- ceinture un espace intérieur (In1) et
- est agencée pour recevoir, dans l'espace intérieur (In1) de ladite unité collectrice , des sécrétions (S) évacuées par aspiration, sachant que le raccord (4) situé côté patient permet l'instauration d'une liaison fluidique, côté patient, entre une unité d'accouplement (6, 7) située côté patient, à l'extérieur de l'unité collectrice (2, 3), et l'espace intérieur (In1) de ladite unité collectrice,
sachant que le canal (22)
- traverse la paroi (2) de part en part et
- permet l'instauration d'une liaison fluidique supplémentaire entre un point de raccordement accessible de l'extérieur, en particulier un raccord (11) situé côté appareillage, et ledit espace intérieur (In1) de l'unité collectrice, et
sachant que le filtre (20) à liquides est conçu pour aspirer au moins une partie d'un liquide, montant dans l'enceinte de l'espace intérieur (In1) de ladite unité collectrice, dès que ce liquide atteint ledit filtre (20) à liquides, et
sachant que le filtre (21) à agents pathogènes
- est conçu pour trier, par filtrage, des agents microbiens pathogènes et des gouttelettes de liquide émanant d'un fluide s'écoulant hors de l'espace intérieur (In1) de l'unité collectrice, avant que ces derniers n'atteignent le point de raccordement (11) et
- inclut au moins un non-tissé triboélectrique,
ledit filtre (21) à agents pathogènes étant implanté en aval du filtre (20) à liquides - en observant dans la direction (U) de l'écoulement de fluides sortant dudit espace intérieur (In1) de l'unité collectrice -,
le ou chaque non-tissé triboélectrique comprenant deux couches parallèles l'une à l'autre, constituées de deux types de fibres différents,
sachant que ces deux couches
- sont agencées perpendiculairement ou à l'oblique par rapport à la direction de l'écoulement et
- sont imbriquées par denture, de façon telle que l'une des couches pénètre mécaniquement dans l'autre couche, au moins dans une partie de la surface exposée à l'écoulement, et
sachant que l'une desdites couches cède des électrons, et que l'autre couche absorbe des électrons ou attire des électrons.

2. Dispositif de collecte selon la revendication 1,
**caractérisé par le fait que**
le filtre (20) à liquides est monté sur une face intérieure (2.i) de la paroi (2) qui pointe en direction de l'espace intérieur (In1) de l'unité collectrice.

3. Dispositif de collecte selon la revendication 1 ou la revendication 2,
**caractérisé par le fait que**
les deux couches constituées des deux types de fibres différents s'interpénètrent, l'interpénétration étant instaurée par aiguilletage mécanique et
l'aiguilletage impliquant une charge électrique des deux couches dans des sens opposés.

4. Dispositif de collecte selon l'une des revendications précédentes,
**caractérisé par le fait que**
le filtre (20) à liquides inclut un filtre fritté.

5. Dispositif de collecte selon l'une des revendications précédentes,
**caractérisé par le fait que**
le filtre (21) à agents pathogènes est additionnellement pourvu d'un non-tissé soufflé à l'état fondu.

6. Dispositif de collecte selon l'une des revendications précédentes,
**caractérisé par le fait que**
le filtre (20) à liquides est conçu pour aspirer au moins une partie d'un liquide atteignant ledit filtre (20) à liquides dans l'enceinte de l'espace intérieur (In1) de l'unité collectrice et
est additionnellement conçu pour aspirer des gouttes de liquide à partir d'un écoulement de fluide circulant à travers ledit filtre (20) à liquides.

7. Dispositif de collecte selon l'une des revendications précédentes,
**caractérisé par le fait que**
le canal (22) est placé à distance du raccord (4) situé côté patient.

8. Système collecteur comprenant
- un dispositif de collecte conforme à l'une des revendications précédentes,
- un réceptacle enveloppant (1) et
- un raccord (11) situé côté appareillage,
sachant que ledit réceptacle enveloppant (1) reçoit et supporte l'unité collectrice (2, 3, 4),
sachant que ledit raccord (11), situé côté appareillage, permet d'instaurer une liaison fluidique à pression négative entre l'espace intérieur (In1) de ladite unité collectrice et une source de dépression (8), à l'extérieur dudit réceptacle enveloppant (1), et
sachant que ladite liaison fluidique à pression négative parcourt le canal (22).

9. Dispositif d'évacuation par aspiration, comprenant
- une source de dépression (8),
- une unité d'accouplement (6, 7) située côté patient et
- un système collecteur conforme à la revendication 8,
ladite unité d'accouplement (6, 7) située côté patient étant reliée, avec étanchéité aux fluides, au raccord (4) situé côté patient,
ladite source de dépression (8) étant reliée, avec étanchéité aux fluides, au raccord (11), situé côté appareillage,
une liaison fluidique, située côté patient,
- étant, ou pouvant être instaurée entre ladite unité d'accouplement (6, 7) située côté patient et l'espace intérieur (In1) de l'unité collectrice et
- parcourant ledit raccord (4) situé côté patient, et
sachant qu'une liaison fluidique à pression négative
- est, ou peut être instaurée entre ladite source de dépression (8) et ledit espace intérieur (In1) de l'unité collectrice et
- parcourt ledit raccord (11), situé côté appareillage, et le canal (22).
